# FASCICULE DE BREVET EUROPEEN

(11) **EP 2 772 547 B1**
(45) Date de publication et mention de la délivrance du brevet: **08.07.2015**
(21) Numéro de dépôt: 14156910.3
(22) Date de dépôt: 27.02.2014
(51) Int. Cl.: C12P 41/00, C12P 7/40, C07C 62/34, C07C 51/08, C07D 223/16

(54) **Procédé de synthèse enzymatique de l'acide (7S) 3,4-dimethoxybicyclo[4.2.0]octa-1,3,5-triene 7-carboxylique et application à la synthèse de l'ivabradine et de ses sels**
Enzymatisches Syntheseverfahren der (7S)-3,4-Dimethoxybicyclo[4.2.0]octa-1,3,5-trien-7-Carbonsäure, und Anwendung bei der Synthese von Ivabradin und seinen Salzen
Method for enzymatic synthesis of (7S)-3,4-dimethoxybicyclo[4.2.0]octa-1,3,5-triene-7-carboxylic acid, and use for the synthesis of ivabradine and the salts thereof

(30) Priorité: 28.02.2013 FR 1351785
(43) Date de publication de la demande: 03.09.2014
(73) Titulaire: Les Laboratoires Servier, 92284 Suresnes Cedex (FR)
(72) Inventeur: Pedragosa Moreau, Sandrine, 45100 ORLEANS (FR); Lefoulon, François, 45000 ORLEANS (FR)

(56) Documents cités:
- WO-A1-2011/138625
- WO-A2-2007/071578
- DE-A1- 10 010 149
- GRADLEY MICHELLE L ET AL: "Asymmetric hydrolysis of R-(-),S(+)-2-methylbutyronitrile by Rhodococcus rhodochrous NCIMB 11216", ARCHIVES OF MICROBIOLOGY, SPRINGER, DE, vol. 161, no. 3, 1 mars 1994 (1994-03-01), pages 246-251, XP008165358, ISSN: 0302-8933, DOI: 10.1007/BF00248700
- ALISON J HOYLE ET AL: "The nitrilases of Rhodococcus rhodochrous NCIMB 11216", ENZYME AND MICROBIAL TECHNOLOGY, vol. 23, no. 7-8, 1 novembre 1998 (1998-11-01), pages 475-482, XP055083723, ISSN: 0141-0229, DOI: 10.1016/S0141-0229(98)00076-3

## Description

La présente invention concerne un procédé de synthèse enzymatique du composé de formule (I): 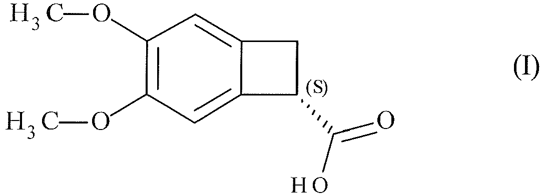 ainsi que son application à la synthèse de l'ivabradine de formule (II) : 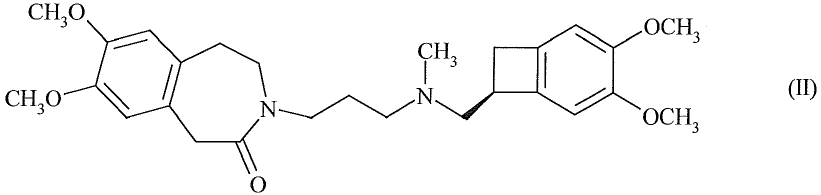 ou 3-{3-[{[(7S)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one,
de ses sels d'addition à un acide pharmaceutiquement acceptable et de leurs hydrates.

L'ivabradine, ainsi que ses sels d'addition à un acide pharmaceutiquement acceptable, et plus particulièrement son chlorhydrate, possèdent des propriétés pharmacologiques et thérapeutiques très intéressantes, notamment des propriétés bradycardisantes, qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.

La préparation et l'utilisation en thérapeutique de l'ivabradine et de ses sels d'addition à un acide pharmaceutiquement acceptable, et plus particulièrement de son chlorhydrate, ont été décrits dans le brevet européen EP 0 534 859.

Ce brevet décrit la synthèse du chlorhydrate de l'ivabradine à partir du composé de formule (III), la (7S)-1-(3,4-diméthoxy bicyclo[4.2.0] octa-1,3,5-triène 7-yl) N-méthyl méthanamine :

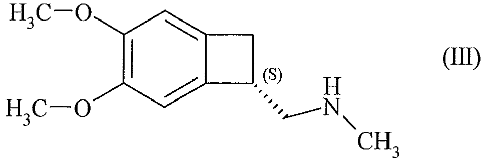

Le composé de formule (III) est un intermédiaire-clé dans la synthèse de l'ivabradine et de ses sels pharmaceutiquement acceptables.

L'art antérieur divulgue plusieurs méthodes d'obtention du composé de formule (III).

Le brevet EP 0 534 859 décrit la synthèse du composé de formule (III) par réduction du nitrile de formule (IV) : 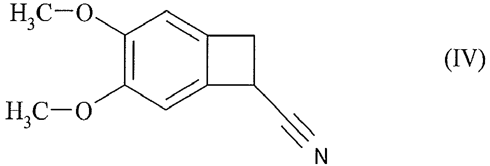 par BH₃ dans le tétrahydrofurane,
suivie de l'addition d'acide chlorhydrique, pour conduire au chlorhydrate de l'amine racémique de formule (V) : 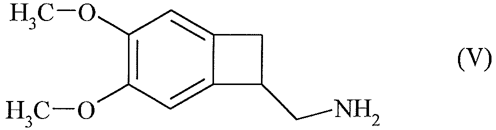 qui est mis en réaction avec du chloroformiate d'éthyle pour conduire au carbamate de formule (VI) : 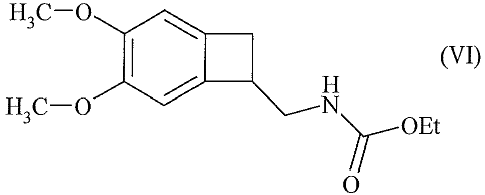 dont la réduction par LiAlH₄ conduit à l'amine méthylée racémique de formule (VII) : 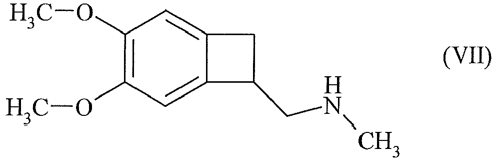 dont le dédoublement à l'aide d'acide camphosulfonique conduit au composé de formule (III). Cette méthode a l'inconvénient de ne conduire au composé de formule (III) qu'avec un rendement très faible de 2 à 3% à partir du nitrile racémique de formule (IV).

Ce rendement très faible est dû au faible rendement (4 à 5%) de l'étape de dédoublement de l'amine secondaire de formule (VII).

Le brevet EP 2 166 004 décrit l'obtention du composé de formule (III) par résolution optique du nitrile racémique de formule (IV) par chromatographie chirale, pour conduire au nitrile optiquement pur de formule (IX) : 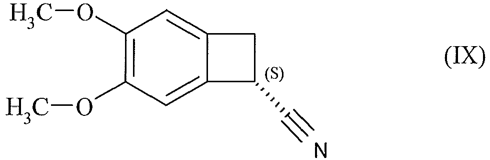 lequel est réduit par NaBH₄ ou par hydrogénation catalytique, pour conduire à l'amine primaire de formule (VIII).

L'amine primaire peut ensuite être méthylée par la même séquence réactionnelle que précédemment (transformation en carbamate, puis réduction).

Le composé de formule (III) peut ainsi être obtenu en 5 étapes à partir du nitrile racémique de formule (IV), avec un rendement de 45,6 % pour l'étape de dédoublement.

Le brevet WO2011138625 décrit un procédé de préparation d'ivabridine dans lequel l'intermédiaire de formule (I) est obtenu par hydrolyse chimique du nitrile racémique de formule (IV), suivi par dédoublement à l'aide d'une base chirale.

Le brevet DE10010149 décrit un procédé d'hydrolyse énantiosélective de nitriles pour obtenir des acides carboxyliques correspondants à l'aide d'une nitrilase du microorganisme Rhodococcus rhodocrous NCIMB 11216.

L'utilisation d'enzymes hydrolytiques nitrilases (EC 3.5.5.1 dans la classification internationale des enzymes) paraissait intéressante pour permettre l'obtention directe de l'acide optiquement pur de formule (I) à partir du nitrile racémique de formule (IV), et réduire ainsi le nombre d'étapes pour l'obtention de l'amine méthylée de formule (III) à partir du nitrile racémique.

Le nitrile de formule (X) a été décrit comme substrat de nitrilases du kit de screening NESK-1400 commercialisé par la société Almac :

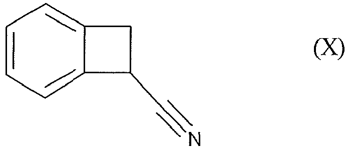

Cependant, l'utilisation de ces mêmes nitrilases sur le nitrile de formule (IV) (cf Exemple comparatif A) a montré une activité faible et peu sélective de ces dernières, conduisant, dans la plupart des cas, à la formation simultanée d'amide (activité nitrile hydratase) et d'acide, difficilement exploitable à des fins de synthèse pour l'obtention d'intermédiaires dans la synthèse du composé de formule (III).

Le problème de la présente invention était donc de trouver une nitrilase permettant la synthèse énantiosélective de l'acide optiquement pur de formule (I) à partir du nitrile racémique de formule (IV), en minimisant la formation d'amide.

Le Demandeur a alors mis en évidence une activité nitrilase de certains microorganismes entiers en faveur de la formation de l'acide de formule (I), de configuration *S.* Sur les microorganismes testés, seul *Rhodococcus rhodocrous* a permis d'obtenir l'acide (S) avec une très bonne énantiosélectivité, sans formation d'amide (cf Exemple comparatif B).
Cette activité a été améliorée par surexpression de la nitrilase.

De façon surprenante, l'hydrolyse enzymatique avec cette nitrilase surexprimée n'est pas énantiosélective sur le substrat de formule (X) (cf Exemple comparatif C).

Plus spécifiquement, la présente invention concerne un procédé de synthèse du composé de formule (I) optiquement pur : 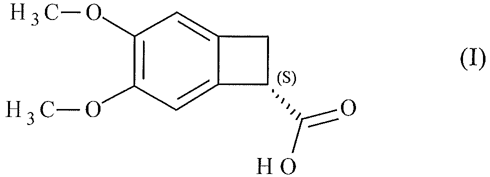 par hydrolyse enzymatique énantiosélective du nitrile racémique ou non optiquement pur de formule (IV) : 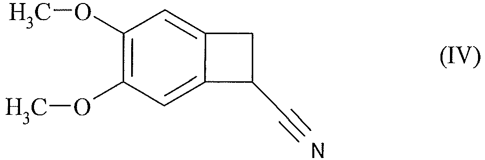 à l'aide de la nitrilase de *Rhodococcus rhodochrous* NCIMB 11216 surexprimée au sein d'un autre organisme possédant un système biologique compétent tel qu'une bactérie, une levure ou un champignon,
dans un mélange d'un solvant organique et d'une solution aqueuse à pH de 5 à 10, préférentiellement un tampon à pH de 5 à 10,
à une concentration de 1 à 500 g/L, préférentiellement de 2 g à 100 g de nitrile de formule (IV) par litre de mélange de solvants,
à un ratio E/S de 1/1 à 1/100,
à une température de 25°C à 40°C.

Selon un aspect de l'invention, la nitrilase est surexprimée au sein d'une bactérie contenant un plasmide réarrangé, telle que *Escherichia coli,* préférentiellement *E. coli* BL21(DE3), *E. coli* BL21(DE3)pLysS, *E. coli* BL21star(DE3) ou *E. coli* JM9(DE3).

Selon un aspect de l'invention, le solvant organique est un solvant miscible à l'eau en totalité ou en partie, tel que le diméthylsulfoxyde, le DMF, l'acétone, l'acétonitrile, un alcool tel que l'éthanol ou l'isopropanol, un éther tel que le THF ou le MTBE.

Selon un autre aspect de l'invention, le solvant organique n'est pas miscible à l'eau, par exemple un hydrocarbure tel que l'heptane ou l'octane.

La solution aqueuse est préférentiellement une solution tampon à pH environ 7.

Selon un aspect de l'invention, les bactéries surexprimant la nitrilase sont utilisées directement dans le procédé, sous forme de culot bactérien ou de lyophilisat.
Le ratio E/S est préférentiellement de 1/1 à 1/10 dans le cas d'un culot bactérien, et de 1/10 à 1/20 dans le cas d'un lyophilisat.

Selon un autre aspect de l'invention, la nitrilase est utilisée sous la forme d'enzyme purifiée.

Le schéma de l'hydrolyse enzymatique selon l'invention est le suivant :

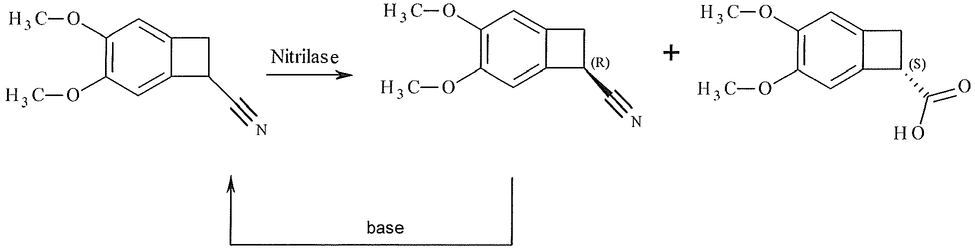

De façon avantageuse, le nitrile de configuration (R), produit secondaire de la réaction, est racémisé par action d'une base organique telle que le DBU ou d'une base minérale telle que la soude, la potasse, le carbonate de potassium ou le carbonate de sodium, pour être recyclé dans le processus d'hydrolyse enzymatique.
Lorsque l'étape de racémisation est effectuée *in situ,* le procédé selon l'invention est un procédé de dédoublement cinétique dynamique (DKR) qui permet d'obtenir l'acide S de formule (I) avec un ee supérieur à 98 %.

L'acide de formule (I) est préférentiellement isolé du milieu réactionnel après un ou plusieurs cycles d'hydrolyse enzymatique.

### Définitions

Par composé optiquement pur, on entend composé dont l'excès énantiomérique est supérieur ou égal à 90%.

Par nitrile non optiquement pur, on entend nitrile dont l'excès énantiomérique est inférieur à 90%.

Par nitrile racémique, on entend nitrile sous la forme d'un mélange de deux énantiomères dans un rapport 55:45 à 45:55.

Par hydrolyse énantiosélective d'un nitrile racémique ou non optiquement pur, on entend hydrolyse préférentielle de l'un des énantiomères du mélange.

Par système biologique compétent, on entend espèce (cellules hôtes) biologique(s) dont le matériel génétique a été modifié par recombinaison génétique la (les) rendant capable(s) de produire une protéine recombinante d'intérêt. Un vecteur d'expression (plasmide) construit à cet effet permet le transfert de l'ADN codant le gène d'intérêt dans la cellule hôte qui peut ainsi (sur)exprimer de manière efficiente la protéine fonctionnelle.

Un autre aspect de l'invention concerne un procédé de synthèse du composé de formule (III) en seulement deux étapes à partir de l'acide optiquement pur de formule (I), qui est transformé en l'amide optiquement pur de formule (XI) : 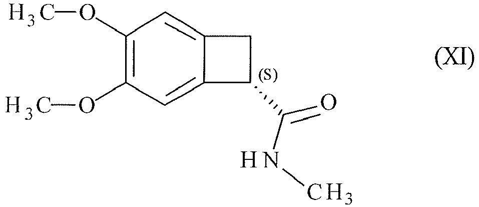 dont la réduction, préférentiellement par BH₃, NaBH₄ ou LiAlH₄, conduit au composé de formule (III).

Le composé de formule (III) est ensuite couplé, soit avec un composé de formule (XII) : 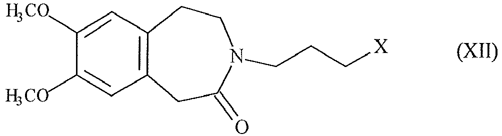 où X représente un atome d'halogène, préférentiellement un atome d'iode,
soit soumis à une réaction d'amination réductrice avec un composé de formule (XIII) en présence d'un agent réducteur : 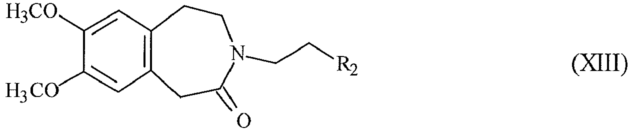 où R₂ représente un groupement choisi parmi CHO et CHR₃R₄,
où R₃ et R₄ représentent chacun un groupement alkoxy (C₁-C₆) linéaire ou ramifié, ou bien forment ensemble avec l'atome de carbone qui les porte un cycle 1,3-dioxane, 1,3-dioxolane ou 1,3-dioxépane,
pour conduire à l'ivabradine, qui est ensuite transformée en un sel d'addition à un acide pharmaceutiquement acceptable, ledit sel étant sous forme anhydre ou hydrate.

Le composé de formule (III) peut être également engagé dans la réaction d'amination réductrice sous la forme de son sel d'addition à un acide pharmaceutiquement acceptable, préférentiellement son chlorhydrate. Dans ce cas, l'ivabradine est obtenue directement sous la forme de chlorhydrate.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique.

Parmi les agents réducteurs utilisables pour la réaction d'amination réductrice entre le composé de formule (III) et le composé de formule (XIII), on peut citer à titre non limitatif les composés donneurs d'hydrure tel que le triacétoxyborohydrure de sodium ou le cyanoborohydrure de sodium, et le dihydrogène en présence d'un catalyseur tel que le palladium, le platine, le nickel, le ruthénium, le rhodium ou un de leurs dérivés, notamment sous forme supportée ou sous forme d'oxydes.
L'agent réducteur préféré pour la réaction d'amination réductrice entre le composé de formule (III) et le composé de formule (XIII) est le dihydrogène catalysé par le palladium sur charbon.

Les exemples ci-dessous illustrent l'invention.

### Abréviations

- ATFA: Acide TriFluoroAcétique
- CCM: Chromatographie sur Couche Mince
- DBU: DiazaBicycloUndécène
- DKR: Dynamic Kinetic Resolution (Dédoublement cinétique dynamique)
- DMF: DiMéthylFormamide
- DMSO: DiMéthylSulfOxyde
- DO: Densité Optique
- E: Coefficient d'énantiosélectivité
- ee: excès énantiomérique
- éq: équivalent molaire
- HPLC: High Performance Liquid Chromatography (Chromatographie en phase liquide à haute performance)
- IPTG: IsoPropyl β-D-1-ThioGalactopyranoside
- LB: milieu de culture Lysogeny Broth
- MeOH: Méthanol
- MTBE: MéthylTertButyl Ether
- po: pureté optique ou énantiomérique
- ratio E/S: ratio Enzyme/Substrat, exprimé en g/g
- RMN: (Spectroscopie) Résonance Magnétique Nucléaire
- SM: Spectrométrie de Masse
- THF: TétraHydroFurane
- TMS: TétraMéthylSilane

### EXEMPLE 1 : (7S)- Acide 3,4-diméthoxybicyclo[4.2.0]octa-1,3,5-triène-7-carboxylique

### Surexpression de la nitrilase :

La protéine nitrilase de *Rhodococcus rhodochrous* NCIMB 11216 est décrite dans les bases de données protéiques et génomiques. La séquence du gène recherché est répertoriée sous l'identifiant SVA (Sequence Version Archive) « EF467367 » au sein de l'ENA (European Nucleotide Archive) de l'EMBL-Bank. Cette séquence correspond à la référence « A4LA85 » de la base de données UniProtKB/TrEMBL.
La souche de production *E. coli* BL21(DE3), transformée avec le vecteur d'expression pET28a-Nit1, a été utilisée.
Le protocole de surexpression de la nitrilase est décrit dans Applied Biochemistry and Biotechnology 2010, Vol 160(2), pp 393-400.
Les cellules ainsi transformées sont soit utilisées directement sous forme de culot bactérien, soit lyophilisées avant utilisation.

### Hydrolyse enzymatique avec la nitrilase surexprimée.

Les cellules transformées selon le protocole ci-dessus sont mises sous agitation à une concentration de 5,6 10⁹ cellule/mL (*1mL de culture à DO*=*1 (600 nm) correspond à 1.10⁹ bactéries et environ 10 mg de culot bactérie ou 1,5 mg de lyophilisat).*
A une solution de 250 mL de tampon phosphate KH₂PO₄/ Na₂HPO₄ 1/15 M à pH 7 sont ajoutés 1g de lyophilisat d'*E*. *coli* et 500 mg (c=2 g/L, 10mM) de 3,4-diméthoxybicyclo[4.2.0] octa-1,3,5-triène-7-carbonitrile dans 2% de DMSO (5 mL).
Le milieu réactionnel est maintenu à 30°C, sous agitation 220 tr/min rotative pendant 6h.

La réaction est contrôlée par HPLC sur phase chirale dans des conditions permettant de déterminer l'excès énantiomérique de l'acide et du nitrile :

*Colonne chiralpak IB*
*90 % n-hexane 10 % 2-PrOH + 0,1 % d'ATFA*
*1mL*/*min 30 °C 288 nm*

| | **% nitrile** | **ee (nitrile)** | **% acide** | **ee (acide)** | **conversion** | **E** |
|---|---|---|---|---|---|---|
| **6h** | 49,9 | 94 | 50,1 | 97 | 0,49 | >100 |

| | | | | | | |
|---|---|---|---|---|---|---|
| * coefficient d'énantiosélectivité E = ln[1-c(1+ee(acide))] / ln[I-c(1-ee(acide))] | | | | | | |

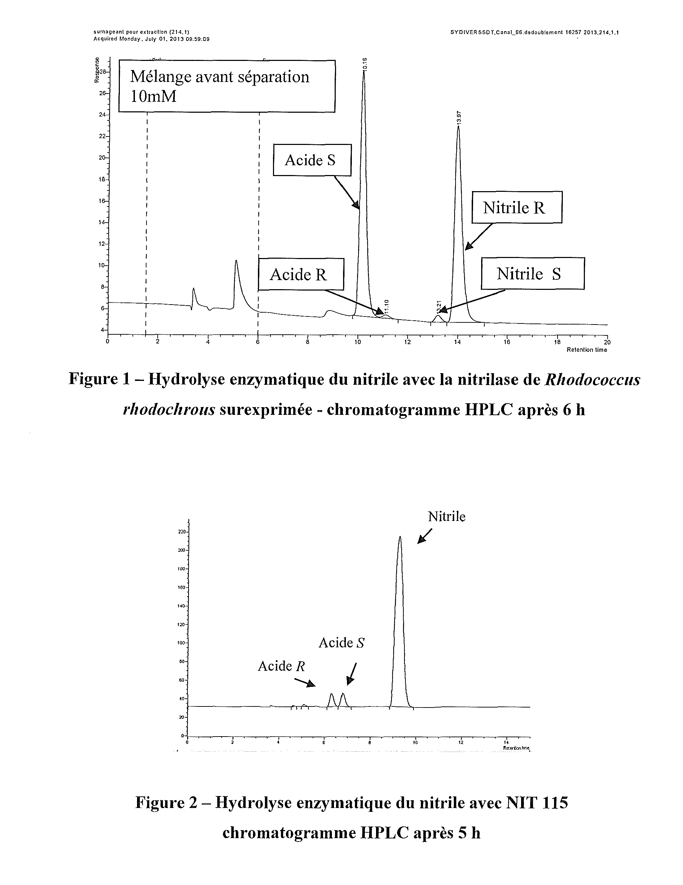

Le chromatogramme HPLC sur phase chirale après 6h est représenté en **Figure 1****.**

Après 6h de réaction, le milieu réactionnel est acidifié par HCl 1M afin d'obtenir un pH très acide (pH 2), puis extrait par 2x100mL de dichlorométhane. La phase organique est soutirée. Une deuxième extraction au toluène (2x100mL) permet de récupérer tout le produit restant dans la phase aqueuse. Les phases organiques sont lavées par une solution de NaCl saturée puis séchées avec du sulfate de magnésium anhydre. Après évaporation des solvants, on obtient le composé brut qui est purifié par chromatographie flash sur colonne de silice dans les conditions suivantes :

*Type de colonne : 80g SiOH Macherey-Nagel*
*Matériel et méthode: Reveleris®*
*Eluant: Isocratique (cyclohexane* +*1% d'acide acétique* / *acétate d'éthyle* +*1% d'acide acétique 75*/*25)*
*Détection : UV 288 nm*
*Débit : 60ml*/*min*

### Résultat :

Nitrile (R) : rendement 36 % (179 mg), ee (R) : 96%
**Acide** (**S**) **: rendement 39 % (246 mg), ee (S) 96%**

### EXEMPLE 2 : 3,4-diméthoxybicyclo[4.2.0] octa-1,3,5-triène-7-carbonitrile par racémisation du nitrile (R)

Dans un ballon muni d'un réfrigérant et d'une agitation magnétique, charger 100 mg du (R)-(3,4-diméthoxy-bicyclo[4.2.0]octa-1,3,5-trién-7-yl)nitrile (0,53 mmol), 5 mL d'isopropanol et 121 mg de DBU (1,5 éq.). Chauffer 2h à 65°C puis laisser revenir à température ambiante. Filtrer pour obtenir le composé du titre.

### EXEMPLE 3 : (7S)-3,4-Diméthoxy-N-méthylbicyclo[4.2.0] octa-1,3,5-triène-7-carboxamide

Mettre en suspension l'acide (7*S*)-3,4-diméthoxybicyclo[4.2.0] octa-1,3,5-triène-7-carboxylique obtenu à l'exemple 1 (300 mg) dans le THF (3 ml) à température ambiante puis ajouter la triéthylamine (200 µl). Le chloroformiate d'éthyle (150 µl) est ajouté lentement au milieu. Le milieu réactionnel précipite (milieu I).
Dans un autre flacon, la méthylamine en solution 2M dans le THF (2,25 ml) est mise sous agitation avec l'eau (1 ml) et la triéthylamine (300 µl). L'agitation est maintenue pendant 20 min puis ce milieu est ensuite additionné au milieu I et laissé sous agitation à température ambiante pendant une nuit.
Le mélange réactionnel est ensuite évaporé et purifié par HPLC préparative.
Le (7*S*)-3,4-diméthoxy-N-méthylbicyclo[4.2.0] octa-1,3,5-triène-7-carboxamide est obtenu avec un rendement de 60%.

¹H RMN (DMSO-d6, ppm / TMS) = 2,61 (m; 3H); 3,16 (m; 2H); 3,71 (s; 6H); 4,05 (m; 1H); 6,78 (s; 1H); 6,81 (s;1H); 7,78 (sl;1H).

### EXEMPLE 4 : (7S)-3,4-Diméthoxybicyclo[4.2.0]octa-1,3,5-trién-7-yl]-N-méthyl-méthanamine

Mettre en suspension le (7*S*)-3,4-diméthoxy-N-méthylbicyclo[4.2.0] octa-1,3,5-triène-7-carboxamide obtenu à l'Exemple 3 (450 mg) dans le tétrahydrofurane (20 mL) puis ajouter lentement 1,6 mL d'une solution de LiAlH₄ 2M dans le tétrahydrofurane au milieu réactionnel à température ambiante. On observe un fort dégagement gazeux et le milieu réactionnel devient limpide. Chauffer le milieu réactionnel à reflux pendant 30 min.
Hydrolyser après le retour à température ambiante, puis extraire par l'acétate d'éthyle. Sécher sur MgSO₄ puis évaporer. Le résidu obtenu est purifié par HPLC préparative (éluant : eau/acétonitrile/acide trifluoroacétique de 98/2/0,2 à 20/80/0,2) en 30 minutes pour conduire au produit du titre avec un rendement de 46%.

¹H RMN (DMSO-d6, ppm / TMS) = 2,60 (m; 3H); 2,85 (m; 1H); 3,15 (m; 1H); 3,25 (dd; 1H); 3,30 (m; 1H); 3,62 (m; 1H); 3,70 (s; 6H); 6,82 (s; 1H); 6,89 (s;1H); 8,48 (sl; 1H).

### EXEMPLE 5 : (7S)-3,4-Diméthoxybicyclo[4.2.0]octa-1,3,5-trién-7-yl]-N-méthyl-méthanamine, chlorhydrate

20 mL d'une solution molaire de BH₃ dans le tétrahydrofurane sont ajoutés à température ambiante au mélange de 2,2 g (10 mmol) du (7*S*)-3,4-diméthoxy-N-méthylbicyclo[4.2.0] octa-1,3,5-triène-7-carboxamide obtenu à l'Exemple 3 dans 45mL de tétrahydrofurane. Après 1h sous agitation, 10mL de la solution de BH₃ dans le tétrahydrofurane sont ajoutés. Après une nuit d'agitation à température ambiante, 20 mL d'éthanol sont additionnés goutte à goutte et le mélange est agité jusqu'à fin de dégagement gazeux (1h environ). 20 mL d'une solution d'acide chlorhydrique dans l'éthanol sont ensuite additionnés goutte à goutte. Après 4h sous agitation, le précipité obtenu (1,2 g de produit du titre) est filtré. Le filtrat est concentré et 0,65g supplémentaires de produit du titre sont obtenus par concrétisation dans un mélange acétate d'éthyle/éthanol 80/20.
Les deux précipités sont réunis pour conduire à 1,85 g du produit du titre (Rendement 77%).

### EXEMPLE 6 : Chlorhydrate de l'ivabradine

Dans un autoclave, charger 5,5 kg de 3-[2-(1,3-dioxolan-2-yl)éthyl]-7,8-diméthoxy-1,3-dihydro-2*H*-3-benzazépin-2-one, 27,5 1 d'éthanol et 550 g de palladium sur charbon.
Purger à l'azote puis à l'hydrogène, chauffer à 55°C, puis hydrogéner à cette température sous une pression de 5 bars jusqu'à absorption de la quantité théorique d'hydrogène.
Ramener ensuite à température ambiante, puis décompresser l'autoclave.
Ajouter ensuite 4 kg du chlorhydrate de la (7S)-3,4-diméthoxybicyclo[4.2.0]octa-1,3,5-trién-7-yl]-N-méthylméthanamine, 111 d'éthanol, 5,5 1 d'eau et 1 kg de palladium sur charbon. Purger à l'azote puis à l'hydrogène, chauffer à 85°C, puis hydrogéner à cette température sous une pression de 30 bars jusqu'à absorption de la quantité théorique d'hydrogène.
Revenir ensuite à température ambiante, purger l'autoclave, puis filtrer le mélange réactionnel, distiller les solvants puis isoler le chlorhydrate d'ivabradine par cristallisation dans un mélange toluène/1-méthyl-2-pyrrolidinone.

Le chlorhydrate d'ivabradine est ainsi obtenu avec un rendement de 85 % et une pureté chimique supérieure à 99 %.

### EXEMPLE comparatif A: Criblage de nitrilases commerciales pour l'hydrolyse enzymatique du 3,4-diméthoxybicyclo[4.2.0]octa-1,3,5-triène-7-carbonitrile

Dans un tube, peser la nitrilase étudiée sous forme de lyophilisat (15 mg) puis ajouter 4 ml de tampon 0,1 M KH₂PO₄ à pH=7 et le 3,4-diméthoxybicyclo[4.2.0] octa-1,3,5-triène-7-carbonitrile (20 mg) solubilisé dans 100µl de DMSO.
Mettre dans l'incubateur à 28°C et 220tr/min.
Le taux de conversion a été mesuré par HPLC au bout de 24 h et 72h.

Les nitrilases NIT 101, NIT 102, NIT 103, NIT 104, NIT 105, NIT 106, NIT 108, NIT 109, NIT 111, NIT 112 et NIT 113 (Almac) n'hydrolysent pas le nitrile après 24h (pas de formation d'acide ni amide).

Les résultats obtenus avec les nitrilases NIT 107, NIT 110, NIT 114 et NIT 115 (Almac) sont résumés dans le tableau ci-dessous :

| Nitrilase | 72h | | |
|---|---|---|---|
| | Amide | Acide | Nitrile |
| NIT 107 | 23% | 16% | 61% |
| NIT 110 | 24% | 15% | 61% |
| NIT 114 | 21% | 22% | 57% |
| NIT 115 | 7% | 47% | 46% |

### Conditions analytiques:

*colonne phenomenex LUNA HST 50***3 C18(2) 2,5µm*
*0% à 100% de B en 8min 0,8ml*/*min 40°C*
*A (1000 eau*+*25 ACN*+*1 ATFA)*
*B (1000 ACN*+*25 eau+1 ATFA)*

La nitrilase NIT 115 a alors été engagée dans un autre essai pour déterminer si l'hydrolyse du nitrile est énantiosélective.

La nitrilase NIT 115 (12 mg ; Almac) a été utilisée dans 6 mL [2 mg/mL] de tampon.
Le 3,4-diméthoxybicyclo[4.2.0] octa-1,3,5-triène-7-carbonitrile a été ajouté pour atteindre une concentration finale de 4 mg/mL en ce dernier.
L'énantiosélectivité a été mesurée par HPLC en utilisant les conditions analytiques suivantes :

*colonne chiralpak IC 250***4.6*
*30% éthanol absolu + 0,1%ATFA + 70%heptane + 0,1%ATFA*
*1ml*/*min 30°C 288nm*
*Remarque* : dans ces conditions, les énantiomères de l'acide sont séparés, mais pas ceux du nitrile.
Le chromatogramme obtenu après 5h de réaction est représenté en **Figure 2****.**

Conclusion : aucune énantiosélectivité n'est observée.

### EXEMPLE comparatif B: Criblage de nitrilases de souches bactériennes et fongiques pour l'hydrolyse enzymatique du 3,4-diméthoxybicyclo[4.2.0] octa-1,3,5-triène-7-carbonitrile

Une étude utilisant plusieurs inducteurs bactériens (propionitrile, benzonitrile, 4-bromobenzonitrile) a montré que le propionitrile permettait la meilleure induction de l'activité nitrilase avec le 3,4-diméthoxybicyclo[4.2.0] octa-1,3,5-triène-7-carbonitrile.
Les souches bactériennes ont été induites au propionitrile à 72 mM durant 72h, les cellules ont été reprises dans 50 mL (concentrées 2 fois, conc.10mg/ml en cellules) de tampon phosphate 0,1M KH₂PO₄/K₂HPO₄ à pH=7,3 et le 3,4-diméthoxybicyclo[4.2.0] octa-1,3,5-triène-7-carbonitrile ajouté à la concentration de 10 mM dans 2% de DMSO v/v_{final}.
Les souches fongiques ont été induites au valéronitrile.
L'ensemble des milieux réactionnels est mis sous agitation 220 tr/min à 30°C pour les bactéries et à 27°C pour les champignons et suivi pendant 96H par HPLC sur phase inverse et par HPLC en phase chirale selon les méthodes décrites ci-dessous :

### Analyse en phase inverse

*colonne phenomenex LUNA HST 50*3 C18(2) 2,5µm*
*0% de B à 100% de B en 8min 0.8ml.min 40°C*
*A (1000 eau*+*25 ACN*+*1 ATFA)*
*B (1000 ACN*+*25 eau*+*1 ATFA)*

### Analyse en phase chirale

*colonne chiralpak IC 250***4.6*
*30% éthanol absolu + 0,1%ATFA + 70%heptane + 0,1%ATFA*
*1ml*/*min 30°C 288nm*

Les résultats obtenus sont résumés dans le tableau ci-dessous :

| **MICROORGANISMES** | **% composés formés après 96h** | | |
|---|---|---|---|
| | **Nitrile résiduel** | **amide** | **acide** |
| *Rhodococcus erythropolis* NCIMB 11215 | 23 | 42 | 35 (S) |
| ***Rhodococcus rhodochrous* NCIMB11216** | **65** | / | **35** (***S***) |
| *Rhodococcus rhodochrous* NCIMB 11273 | 100 | / | / |
| *Rhodococcus rhodnii* NCIMB11279 | 100 | / | / |
| *Aspergillus niger* BO | 95 | / | < 5 |
| *Aspergillus alliaceus* NRRL 315 | 95 | / | < 5 |
| *Cunninghamella elegans* NRRL 1392 | 95 | / | < 5 |
| *Rhizopus nigricans* NRRL 1477 | 95 | / | < 5 |
| *Absidia cylindrospora* MMP 1569 | 95 | / | < 5 |
| *Mortierella isabellina* NRRL 1757 | 95 | / | < 5 |
| *Mucor plumbeus* ATCC 4740 | 95 | / | < 5 |
| *Beauveria bassiana* ATCC 7159 | 86 | / | 14 |
| *Stibella fimetaria* CBS 548-84 | 100 | / | / |
| *Stibella fimetaria* CBS 511-67 | 100 | / | / |
| *Stibella fimetaria* CBS 294-81 | 100 | / | / |

### EXEMPLE comparatif C: Hydrolyse enzymatique du bicyclo[4.2.0] octa-1,3>5-triène-7-carbonitrile avec la nitrilase de Rhodococcus rhodochrous NCIMB 11216 surexprimée

Etalement sur des boîtes de LB+agar+kanamycine, incubation en statique à 37°C pendant 24h (Souche 11216 de nitrilase d'E. coli recombinante).
Préculture dans 5ml de LB+kanamycine(50mg/l), incubation à 37°C, 180 tours/min pendant une nuit.
Culture : mettre 50ml de LB et 500µl de préculture dans des fioles d'erlenmeyer non bafflées de 250 ml, incubation à 28°C, 160 tours/min jusqu'à ce que la DO soit égale à 0,6 (soit environ 4h).
Induction à l'IPTG (0,5mM), incubation à 17°C, 160 tours/min pendant une nuit (17 heures). Test d'activité : centrifuger les cultures à 4°C, 6000 tours/min pendant 20 minutes, resuspendre le culot dans 10 ml de tampon phosphate 0,1M pH 7. Ajouter le bicyclo[4.2.0] octa-1,3,5-triène-7-carbonitrile (10mM) +2% d'éthanol. Incuber à 220 tours/mn, 30°C.
*Remarque : si la culture est de plus de 50ml au moment de la centrifugation on prélève 50ml puis on fait le test d'activité avec un culot de 50ml de culture.*

Suivi de l'hydrolyse par chromatographie chirale : à 45 min et 2h.

*Colonne : Phenomenex®LUNA HST 50*3 C18(2) 2.5µm*
*Eluant : A* + *B (de 0% à 100% de B en 8min)*
*A : 1000 eau*+*25 ACN*+*1 ATFA*
*B :1000 ACN*+*25 eau*+*1 ATFA*
*0.8m*/*min - 40°C - UV 210 nm*

### Résultats :

| Temps | Nitrile | Acide carboxylique |
|---|---|---|
| 45 minutes | 50% | 50% |
| 2 heures | 0% | 100 % |

Le suivi par chromatographie chirale montre que la réaction n'est pas énantiosélective.

## Revendications

1. Procédé de synthèse du composé de formule (I) optiquement pur : 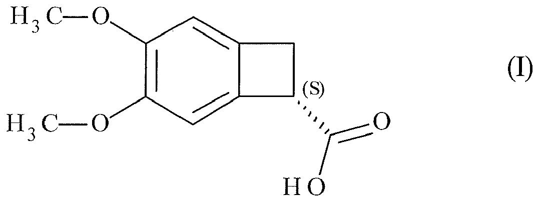 par hydrolyse enzymatique énantiosélective du nitrile racémique ou non optiquement pur de formule (IV) : 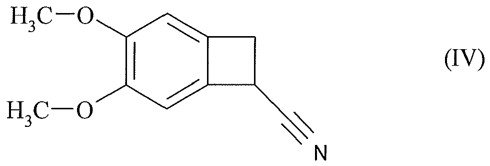 à l'aide de la nitrilase de *Rhodococcus rhodochrous* NCIMB 11216 surexprimée au sein d'un autre organisme possédant un système biologique compétent,
dans un mélange d'un solvant organique et d'une solution aqueuse à pH de 5 à 10,
à une concentration de 1 à 500 g de nitrile de formule (IV) par litre de mélange de solvants,
à un ratio E/S de 1/1 à 1/100,
à une température de 25°C à 40°C.

2. Procédé selon la revendication 1, où l'organisme possédant un système biologique compétent est une bactérie contenant un plasmide réarrangé.

3. Procédé selon la revendication 2, où les bactéries surexprimant la nitrilase sont utilisées directement, sous forme de culot bactérien ou de lyophilisat.

4. Procédé selon l'une quelconque des revendications 1 à 3, où le solvant organique est choisi parmi le diméthylsulfoxyde, le DMF, l'acétone, l'acétonitrile, l'éthanol, l'isopropanol, le THF et le MTBE.

5. Procédé de synthèse selon l'une quelconque des revendications 1 à 4, dans lequel le nitrile de configuration (R), produit secondaire de la réaction : 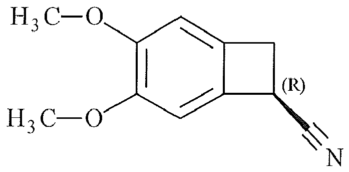 est racémisé par action d'une base en nitrile racémique de formule (IV) pour être recyclé dans le processus d'hydrolyse enzymatique.

6. Procédé de synthèse selon la revendication 5, dans lequel la base est la DBU.

7. Procédé de synthèse selon l'une quelconque des revendications 5 ou 6, dans lequel l'étape de racémisation est effectuée *in situ.*

8. Procédé de synthèse selon l'une quelconque des revendications 5 à 7, dans lequel l'acide de formule (I) est isolé après un ou plusieurs cycles d'hydrolyse enzymatique.

9. Procédé de synthèse du composé de formule (III) : 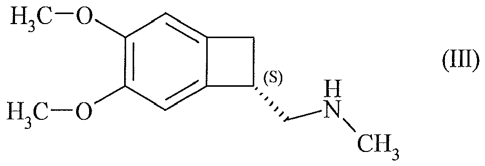 à partir du nitrile de formule (IV) : 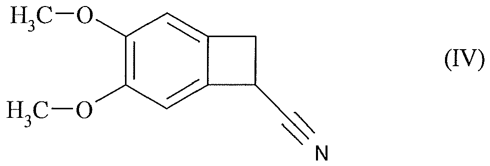 qui est hydrolysé en acide optiquement pur de formule (I) : 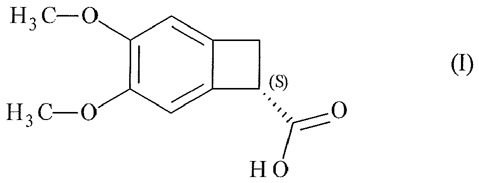 selon l'une quelconque des revendications 1 à 8,
qui est ensuite transformé en l'amide optiquement pur de formule (XI) : 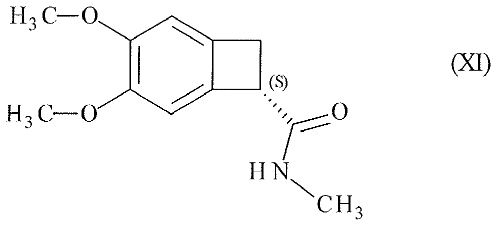 dont la réduction conduit au composé de formule (III).

10. Procédé de synthèse selon la revendication 9, dans lequel la réduction du composé de formule (XI) en composé de formule (III) est effectué par BH₃, NaBH₄ ou LiAlH₄.

11. Procédé de synthèse selon l'une quelconque des revendications 9 ou 10, dans lequel le composé de formule (III) est ensuite soit couplé avec un composé de formule (XII) : 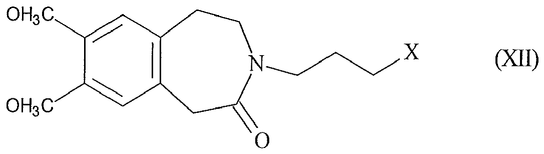 où X représente un atome d'halogène,
soit soumis à une réaction d'amination réductrice avec un composé de formule (XIII) en présence d'un agent réducteur : 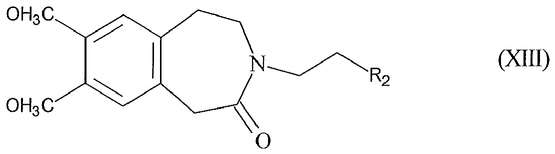 où R₂ représente un groupement choisi parmi CHO et CHR₃R₄,
où R₃ et R₄ représentent chacun un groupement alkoxy (C₁-C₆) linéaire ou ramifié, ou bien forment ensemble avec l'atome de carbone qui les porte un cycle 1,3-dioxane, 1,3-dioxolane ou 1,3-dioxépane,
pour conduire à l'ivabradine, qui est ensuite transformée en un sel d'addition à un acide pharmaceutiquement acceptable sous forme anhydre ou hydrate.

12. Procédé de synthèse selon la revendication 11, dans lequel X est un atome d'iode.

13. Procédé de synthèse selon la revendication 11, dans lequel le composé de formule (III) est engagé dans la réaction d'amination réductrice sous la forme de son chlorhydrate, pour conduire à l'ivabradine sous la forme de chlorhydrate.

14. Procédé de synthèse selon l'une quelconque des revendications 11 ou 13, dans lequel la réaction d'amination réductrice avec le composé de formule (XIII) est effectuée en présence de dihydrogène catalysé par le palladium sur charbon.

## Patentansprüche

1. Verfahren zur Synthese der optisch reinen Verbindung der Formel (I): 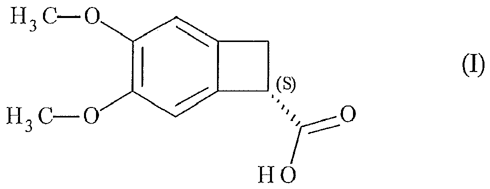 durch enantioselektive enzymatische Hydrolyse des racemischen oder nicht optisch reinen Nitrils der Formel (IV): 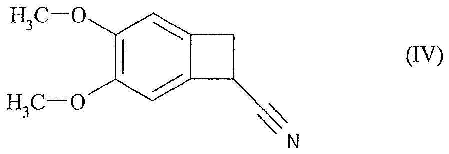 mit Hilfe der Nitrilase von *Rhodococcus rhodochrous* NCIMB 11216, die mit Hilfe eines anderen Organismus, der ein biologisch kompetentes System aufweist, überexprimiert worden ist,
in einer Mischung aus einem organischen Lösungsmittel und einer wässrigen Lösung mit einem pH-Wert von 5 bis 10,
bei einer Konzentration von 1 bis 500 g des Nitrils der Formel (IV) pro Liter der Lösungsmittelmischung,
bei einem E/S-Verhältnis von 1/1 bis 1/100,
bei einer Temperatur von 25 °C bis 40 °C.

2. Verfahren nach Anspruch 1, worin der ein biologisch kompetentes System aufweisende Organismus eine Bakterie ist, die ein umgelagertes Plasmid enthält.

3. Verfahren nach Anspruch 2, worin die die Nitrilase überexprimierenden Bakterien direkt in Form eines Zentrifugenrückstands oder Lyophilisats von Bakterien verwendet werden.

4. Verfahren nach einem der Ansprüche 1 bis 3, worin das organische Lösungsmittel ausgewählt wird aus Dimethylsulfoxid, DMF, Aceton, Acetonitril, Ethanol, Isopropanol, THF und MTBE.

5. Syntheseverfahren nach einem der Ansprüche 1 bis 4, worin das als Nebenprodukt der Reaktion gebildete Nitril der Konfiguration (R): 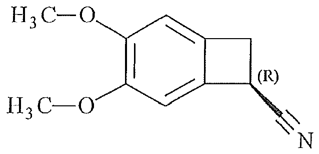 durch Einwirkung einer Base zu dem racemischen Nitril der Formel (IV) racemisiert wird und dann in den enzymatischen Hydrolyseprozess recyclisiert wird.

6. Syntheseverfahren nach Anspruch 5, worin die Base DBU ist.

7. **Syntheseverfahren** nach einem der Ansprüche 5 oder 6, worin die Racemisierungsstufe *in situ* durchgeführt wird.

8. Syntheseverfahren nach einem der Ansprüche 5 bis 7, worin die Säure der Formel (I) nach einem oder mehreren Zyklen der enzymatischen Hydrolyse isoliert wird.

9. Verfahren zur Synthese der Verbindung der Formel (III): 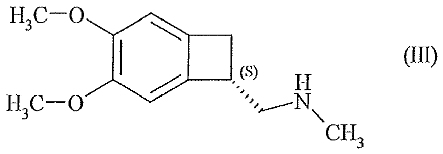 ausgehend von dem Nitril der Formel (IV): 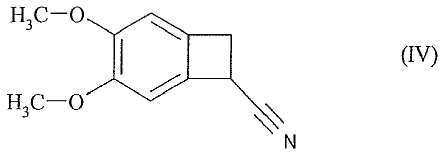 welches zu der optisch reinen Säure der Formel (I): 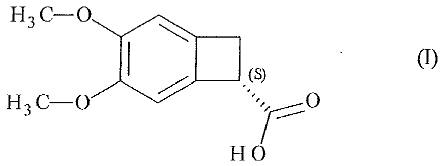 nach einem der Ansprüche 1 bis 8 hydrolysiert wird,
die anschließend in das optisch reine Amid der Formel (XI) überführt wird: 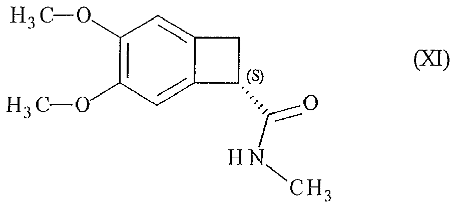 dessen Reduktion zu der Verbindung der Formel (III) führt.

10. Syntheseverfahren nach Anspruch 9, worin die Reduktion der Verbindung der Formel (XI) zu der Verbindung der Formel (III) mit BH₃, NaBH₄ oder LiAlH₄ bewirkt wird.

11. Syntheseverfahren nach einem der Ansprüche 9 oder 10, worin die Verbindung der Formel (III) anschließend entweder mit einer Verbindung der Formel (XII): 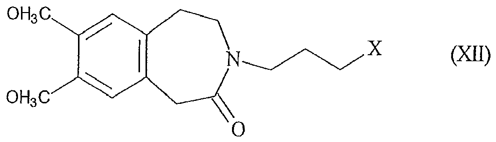 worin X ein Halogenatom bedeutet, gekuppelt wird
oder in Gegenwart eines Reduktionsmittels einer reduzierenden Aminierungsreaktion mit einer Verbindung der Formel (XIII) unterworfen wird: 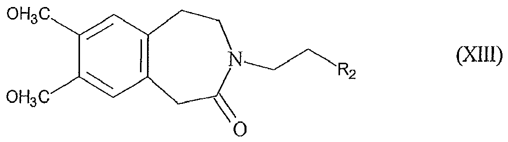 worin R₂ eine Gruppe ausgewählt aus CHO und CHR₃R₄ bedeutet,
worin R₃ und R₄ jeweils eine geradkettige oder verzweigte (C₁-C₆)-Alkoxygruppe bedeuten oder gemeinsam mit dem sie tragenden Kohlenstoffatom einen 1,3-Dioxan-, 1,3-Dioxolan- oder 1,3-Dioxepan-Ring bilden,
zur Bildung von Ivabradin, das anschließend in ein Additionssalz mit einer pharmazeutisch annehmbaren Säure in wasserfreier Form oder in Form des Hydrats umgewandelt wird.

12. Syntheseverfahren nach Anspruch 11, worin X ein Iodatom bedeutet.

13. Syntheseverfahren nach Anspruch 11, worin die Verbindung der Formel (III) in Form ihres Hydrochlorids bei der reduzierenden Aminierungsreaktion eingesetzt wird zur Bildung von Ivabradin in Form des Hydrochlorids.

14. Syntheseverfahren nach einem der Ansprüche 11 oder 13, worin die reduzierende Aminierungsreaktion mit der Verbindung der Formel (XIII) in Gegenwart von mit Palladium-auf-Kohlenstoff katalysiertem Wasserstoff durchgeführt wird.

## Claims

1. Process for the synthesis of the optically pure compound of formula (I): 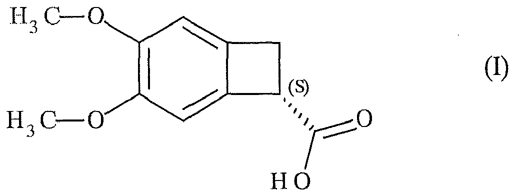 by enantioselective enzymatic hydrolysis of the racemic, or not optically pure, nitrile of formula (IV): 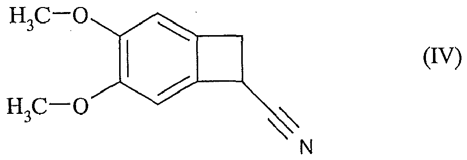 using the nitrilase of *Rhodococcus rhodochrous* NCIMB 11216 over-expressed in another organism having a competent biological system,
in a mixture of an organic solvent and an aqueous solution having a pH from 5 to 10,
at a concentration from 1 to 500 g of nitrile of formula (IV) per litre of solvent mixture,
at an E/S ratio of from 1/1 to 1/100,
at a temperature from 25°C to 40°C.

2. Process according to claim 1, wherein the organism having a competent biological system is a bacteria comprising a rearranged plasmid.

3. Process according to claim 2, wherein the bacteria over-expressing the nitrilase are used directly, in the form of a bacterial slurry or lyophilisate.

4. Process according to any one of claims 1 to 3, wherein the organic solvent is selected from dimethyl sulphoxide, DMF, acetone, acetonitrile, ethanol, isopropanol, THF and MTBE.

5. Synthesis process according to any one of claims 1 to 4, wherein the nitrile of configuration (R), the secondary product of the reaction: 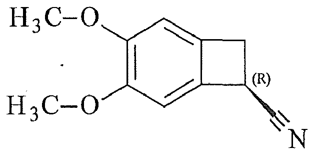 is racemised by the action of a base to form the racemic nitrile of formula (IV) in order to be recycled into the enzymatic hydrolysis process.

6. Synthesis process according to claim 5, wherein the base is DBU.

7. Synthesis process according to either claim 5 or claim 6, wherein the racemisation step is carried out *in situ.*

8. Synthesis process according to any one of claims 5 to 7, wherein the acid of formula (I) is isolated after one or more enzymatic hydrolysis cycles.

9. Process for the synthesis of the compound of formula (III): 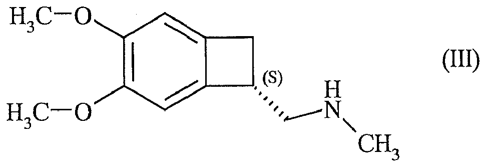 starting from the nitrile of formula (IV): 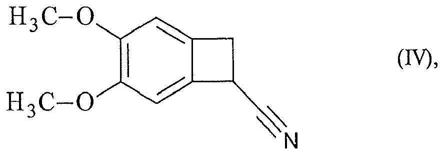 which is hydrolysed to form the optically pure acid of formula (I): 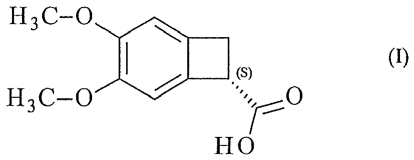 in accordance with any one of claims 1 to 8,
which is then converted into the optically pure amide of formula (XI): 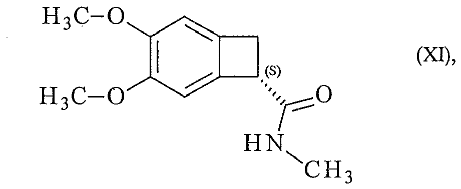 the reduction of which yields the compound of formula (III).

10. Synthesis process according to claim 9, wherein the reduction of the compound of formula (XI) to form the compound of formula (III) is carried out by BH₃, NaBH₄ or LiAlH₄.

11. Synthesis process according to either claim 9 or claim 10, wherein the compound of formula (III) is subsequently either coupled with a compound of formula (XII): 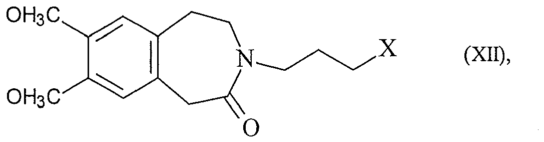 wherein X represents a halogen atom,
or subjected to a reductive amination reaction with a compound of formula (XIII) in the presence of a reducing agent: 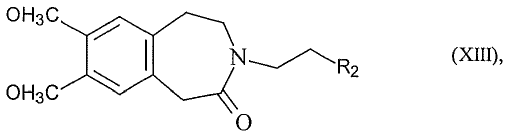 wherein R₂ represents a group selected from CHO and CHR₃R₄,
wherein R₃ and R₄ each represent a linear or branched (C₁-C₆)alkoxy group or form, together with the carbon atom carrying them, a 1,3-dioxane, 1,3-dioxolane or 1,3-dioxepane ring,
to yield ivabradine, which is then converted into an addition salt with a pharmaceutically acceptable acid in anhydrous or hydrate form.

12. Synthesis process according to claim 11, wherein X is an iodine atom.

13. Synthesis process according to claim 11, wherein the compound of formula (III) is used in the reductive amination reaction in the form of its hydrochloride to yield ivabradine in the form of the hydrochloride.

14. Synthesis process according to either claim 11 or claim 13, wherein the reductive amination reaction with the compound of formula (XIII) is carried out in the presence of dihydrogen catalysed by palladium-on-carbon.
